# EUROPÄISCHE PATENTSCHRIFT

(11) **EP 0 790 973 B1**
(45) Veröffentlichungstag und Bekanntmachung des Hinweises auf die Patenterteilung: **29.12.1999**
(21) Anmeldenummer: 95936435.7
(22) Anmeldetag: 08.11.1995
(51) Int. Cl.: C07C 59/255, C07C 51/16

(54) **VERFAHREN ZUR HERSTELLUNG VON WEINSÄURE**
METHOD OF PREPARING TARTARIC ACID
PROCEDE DE PREPARATION D'ACIDE TARTRIQUE

(30) Priorität: 10.11.1994 DE 4440191
(43) Veröffentlichungstag der Anmeldung: 27.08.1997
(73) Patentinhaber: FORSCHUNGSZENTRUM JÜLICH GMBH, 52425 Jülich (DE)
(72) Erfinder: KLASEN, Ralf, D-51373 Leverkusen (DE); SAHM, Hermann, D-52428 Jülich (DE); MATZERATH, Ingo, D-51381 Leverkusen (DE); KLÄUI, Wolfgang, NL-6291 CL Vaals (NL)
(86) Internationale Anmeldenummer: DE9501568
(87) Internationale Veröffentlichungsnummer: WO9615095

(56) Entgegenhaltungen:
- US-A- 2 197 021

## Beschreibung

Die Erfindung bezieht sich auf ein Verfahren zur Herstellung von Weinsäure.

Weinsäure findet vor allem in der Lebensmittel- und Textil-industrie vielfältige Anwendung. So wird Weinsäure beispielsweise als Lebensmittelzusatzstoff, insbesondere als Antioxidations- und Säuerungsmittel, eingesetzt. Darüber hinaus wird Weinsäure als Baustoffadditiv zur Modifizierung des Abbindeverhaltens vor Zement verwendet und dient als Ausgangssubstanz für zahlreiche chemische Reaktionen, insbesondere für chirale Synthesen.

Ein Weg zur Herstellung von Weinsäure ist dessen Gewinnung aus Weinstein, einer Substanz, die bei der Herstellung von Wein anfällt. Allerdings ist der bei der Herstellung von Wein anfallende Rohweinstein in der Regel durch organische Materialien verunreinigt, so daß es aufwendiger Aufarbeitungen bedarf, um aus dem Weinstein die Weinsäure zu erhalten.

Aufgrund dieses Nachteils wurden alternative Verfahren zur Herstellung von Weinsäure entwickelt. So wird beispielsweise in der US-Patentschrift Nr. 1 425 605 die Herstellung von Weinsäure durch Oxidation von Kohlenhydraten in Gegenwart von Nitrat beschrieben. Diese Verfahrensweise führt allerdings zu verschiedenen Stereoisomeren der Weinsäure und ist daher relativ unselektiv.

Darüber hinaus sind aber auch die auf chemischem Wege erreichten Umsätze der eingesetzten Ausgangssubstanzen zu Weinsäure relativ gering: Beispielsweise variiert der Umsatz von 5-Ketogluconat oder anderen Kohlenhydraten zu Weinsäure unter Verwendung von Salpetersäure als Oxidationsmittel von etwa 10 % (US-Patentschtift Nr. 2 380 196) bis maximal etwa 40 % (W.E. Barch, J. Am. Chem. Soc. 55, 3653, 1933). Die Umsetzung von 5-Ketogluconsäure zu Weinsäure in alkalischem Milieu mit 1 M Calciumhydroxid-Lösung bzw. Natronlauge führte zu Weinsäureausbeuten bis etwa 10 % (H. Isbell und N. Holt, J. Res. Bur. Stand. 35, 433, 1945).

Ein weiteres Verfahren zur Herstellung von Weinsäure besteht in der Umsetzung von Glucose zu Weinsäure durch Mikroorganismen, wie Acetobacter oder Gluconobacter, in Gegenwart von Vanadat als Spurenelement (vgl. US-Patentschrift Nr. 3 585 109). Es konnte gezeigt werden, daß bei diesem Verfahren die Weinsäure-Herstellung in zwei Schritten erfolgt: In einem ersten Schritt scheiden die in Glucose-Medium kultivierten Mikroorganismen 5-Ketogluconat in das Medium aus, während in einem zweiten Schritt die Umsetzung von 5-Ketogluconat zu Weinsäure mit dem im Medium als Spurenelement enthaltenen Vanadiumsalz rein chemisch, also unabhängig von der Gegenwart von Mikroorganismen erfolgt (R. Klasen, S. Bringer-Meyer und H. Sahm, Biotechn. and Bioenergin. 40, 183, 1992). Dieses Herstellungsverfahren hat insbesondere den Vorteil, daß nur ein Isomer der Weinsäure, nämlich die gewünschte L- (+)-Weinsäure, entsteht. Allerdings ist der bisher durch dieses Verfahren erreichte Umsatz von Ketogluconat zu Weinsäure noch unbefriedigend.

Die US-Schrift 2 197 021 offenbart ein Verfahren zur Herstellung von d-Weinsäure unter sauren oder alkalischen Bedingungen, bei dem 5-Ketogluconsäure mit sauerstoffhaltigen Gasen ohne Katalysator umgesetzt wird. Die so hergestellte d-Weinsäure ist nebenproduktfrei.

Es ist Aufgabe der Erfindung, ein einfaches Verfahren zur Herstellung von Weinsäure zu schaffen, durch das ein möglichst hoher Umsatz von 5-Ketogluconat zu Weinsäure erreicht wird.

Diese Aufgabe wird erfindungsgemäß dadurch gelöst, daß 5-Ketogluconat in Carbonatpuffer bei einem alkalischen pH-Wert oxidativ zu Weinsäure umgesetzt wird. Es hat sich überraschenderweise gezeigt, daß unter diesen Reaktionsbedingungen, also auch ohne Zugabe von Vanadat oder einem sonstigen Katalysator, eine Umsetzung von 5-Ketogluconat zu Weinsäure erfolgt, wobei ein Umsatz von 5-Ketogluconat zu Weinsäure von bis zu 55% erreicht wird.

Wird dem Reaktionsgemisch zusätzlich Vanadat zugegeben, wird sogar bis zu etwa 75% des eingesetzten 5-Ketogluconats zu Weinsäure umgesetzt. In Gegenwart anderer Puffer, wie beispielsweise alkalischem Boratpuffer, ist die Weinsäurebildung dagegen vollständig gehemmt.

Der pH-Bereich, in dem die Umsetzung vorzugsweise durchgeführt wird, umfaßt den Bereich von pH 8 bis pH 10.

### Ausführungsbeispiel:

Es wurden 2,0 ml einer 0,50 M Lösung des Kaliumsalzes der 5-Ketogluconsäure in 20 ml Meßkolben vorgelegt. Dazu wurden 0,40 ml einer 50 nM Ammoniumvanadatlösung gegeben und mit 0,50 M Cabonat- oder Phosphatpuffer (pH 10,0) auf 20ml aufgefüllt. Analog wurden Lösungen mit 0,40 ml Wasser statt Ammoniumvanadat hergestellt.

Nach Mischen der Komponenten wurde der pH-Wert der Lösungen kontrolliert und anschließend die Reaktionsmischungen in zuvor autoklavierten 50 ml Erlenmeyerkolben steril filtriert. Die Kolben wurden mit Wattestopfen steril verschlossen und in einem Inkubationsschüttler bei 27°C und 180 Upm über acht Tage geschüttelt.

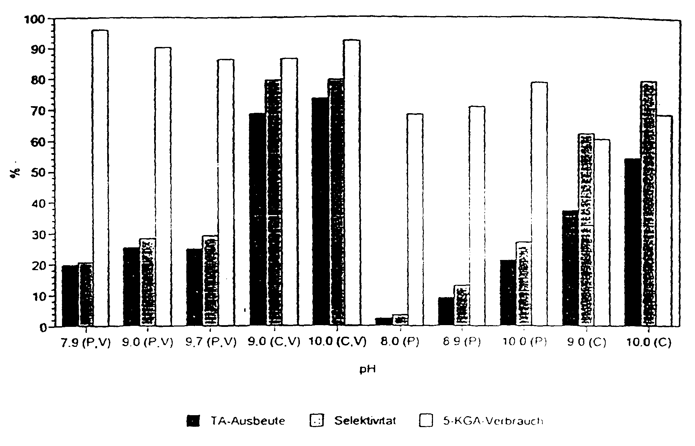

Der Verbrauch an 5-Ketogluconsäure und die gebildete Menge an Weinsäure wurden anschließend mittels HPLC bestimmt. Daraus ließen sich Ausbeute und Selektivität der Reaktion ermitteln. Die Ergebnisse sind in Figur 1 dargestellt.

In Figur 1 bedeuten:
C: Carbonatpuffer, P: Phosphatpuffer, V: Vanadat
TA: Weinsäure, 5-KGA: 5-Ketogluconat
- Ausbeute:: Umsatz des eingesetzten 5-Ketogluconats zu Weinsäure, wobei der Umsatz von 1 Mol 5-Ketogluconsäure zu 1 Mol Weinsäure gleich 100 % gesetzt ist.
- Selektivität:: Umsatz des abgebauten 5-Ketogluconats zu Weinsäure

Wie aus Figur 1 hervorgeht, beträgt die Weinsäureausbeute bei Umsetzung von 5-Ketogluconsäure in Phosphatpuffer mit Vanadat als Katalysator und alkalischem pH-Wert bis zu etwa 25%, dagegen in Carbonatpuffer bis zu etwa 75%. Auch erfolgt eine Umsetzung von 5-Ketogluconat zu Weinsäure ohne Katalysator, wobei eine Weinsäureausbeute in Carbonatpuffer bei pH 10 von etwa 55% erreicht wurde. Weiterhin lag die Selektivität bei Umsetzung des 5-Ketogluconats in Carbonatpuffer mit Werten zwischen etwa 65 bis 80% relativ hoch, was gleichbedeutend mit einer geringen Nebenproduktbildung ist. Damit wird unter den gewählten Reaktionsbedingungen der größte Anteil der abgebauten 5-Ketogluconsäure zu Weinsäure umgesetzt.

## Patentansprüche

1. Verfahren zur Herstellung von Weinsäure, indem 5-Ketogluconat in Carbonatpuffer bei einem alkalischen pH-Wert oxidativ zu Weinsäure umgesetzt wird.

2. Verfahren nach Anspruch 1,
**dadurch gekennzeichnet,**
daß die Umsetzung mit Vanadat durchgeführt wird.

3. Verfahren nach Anspruch 1 oder 2,
**dadurch gekennzeichnet,**
daß die Umsetzung vorzugsweise in einem pH-Bereich von 8 bis 10 durchgeführt wird.

## Claims

1. A method of producing tartaric acid, wherein a 5-ketogluconate is oxidatively converted to tartaric acid in a carbonate buffer at an alkaline pH.

2. A method according to claim 1,
characterised in that
the conversion is effected with vanadate.

3. A method according to claims 1 or 2,
characterised in that
the conversion is preferably effected within a pH range from 8 to 10.

## Revendications

1. Procédé de préparation d'acide tartrique, dans lequel on fait réagir du 5-cétogluconate par oxydation dans un tampon de carbonate à un pH alcalin pour obtenir de l'acide tartrique.

2. Procédé selon la revendication 1, caractérisé en ce qu'on effectue la réaction avec du vanadate.

3. Procédé selon la revendication 1 ou 2, caractérisé en ce qu'on effectue la réaction, de préférence, dans une plage de pH de 8 à 10.
